# DEMANDE DE BREVET EUROPEEN

(11) **EP 3 680 216 A1**
(43) Date de publication de la demande: **15.07.2020**
(21) Numéro de dépôt: 20153900.4
(22) Date de dépôt: 29.03.2017
(51) Int. Cl.: C01B 33/18, A23L 33/16, A61K 8/25, A61K 9/00, C01B 33/193, A23L 29/00, A61K 8/02, A61K 8/60, A61Q 19/00

(54) **SILICES MÉSOPOREUSES ET LEUR PROCÉDÉ DE SYNTHÈSE**

(30) Priorité: 22.04.2016 BE 201605280
(62) Demande divisionnaire de: 17715089.3
(71) Demandeur: Sil'Innov SCRL, 6180 Courcelles (BE)
(72) Inventeur: CROIZET-BERGER, Karine, 1490 Court St Etienne (BE); DELMEULE, Maxim, 6180 Courcelles (BE); ESTAGER, Julien, 2550 Kontich (BE); MANNU, Nicolas, 1410 Waterloo (BE); KARTHEUSER, Benoît, 5590 Ciney (BE)
(74) Mandataire: Brantsandpatents bvba

(57) **Abrégé**

La présente invention fournit une silice mésoporeuse préparée en mélangeant les composants A et B tel que A est un tensioactif de la famille des saponines et B est un précurseur de silice. L'invention fournit aussi une méthode de production de ladite silice.

## Description

### Champ de l'invention

La présente invention concerne une silice mésoporeuse et une méthode de production de ladite silice. Le procédé et la silice sont caractérisés par l'utilisation de composés naturels appartenant à la famille des saponines comme agent de structuration.

### Etat de l'art

Grâce à leur surface spécifique élevée, les matériaux poreux et notamment les silices mésoporeuses présentent un intérêt majeur dans des domaines aussi variés que la catalyse, l'électronique, les semi-conducteurs, les matériaux composites et ils sont aujourd'hui également un outil irremplaçable dans les domaines biologique et médical.

Le mécanisme de synthèse des matériaux silicés mésoporeux organisés est bien connu. Ce mécanisme, dénommé CTM (Cooperative Templating Mechanism ou Mécanisme Coopératif d'Assemblage), propose que l'ajout d'un précurseur de silice conduit, par interaction avec les agrégats formés par un tensioactif, à la formation d'une phase organisée hybride organique/inorganique. Ce mécanisme est donc fondé sur un auto-assemblage entre les molécules tensioactives et le précurseur inorganique qui conduit à une organisation du système. Il existe un vaste panel de tensioactifs pouvant être utilisés comme agents de structuration dans les procédés de synthèse de silice mésoporeuse. Ils peuvent être anioniques, cationiques ou neutres.

Quels que soient les procédés de synthèse mis en œuvre, les tensioactifs sont, dans tous les cas, d'origine non naturelle. Il s'agit de molécules pétrosourcées devant être extraites du matériau final afin, d'une part de permettre une utilisation ultérieure dans des applications médicales, biologiques ou pharmaceutiques et d'autre part, de libérer la porosité. Le brevet EP 2256088 fait usage d'un procédé de calcination afin d'extraire les molécules de tensioactifs. Ce procédé, en plus d'être énergivore détruit le tensioactif utilisé qui, s'il est onéreux ne peut pas entrer dans un processus de recyclage. La calcination présente également l'inconvénient de modifier les propriétés physico-chimiques des matériaux mésoporeux. Ce procédé diminue, par exemple, le paramètre de maille (Bérubé et Kaliaguine. Micropor Mesopor Mat. 2008, 115 : 469-479). Par ailleurs, le caractère exothermique du processus augmente les défauts présents dans la structure inorganique.

Il existe d'autres techniques permettant d'extraire le tensioactif du matériau mésoporeux. On peut citer un procédé d'extraction par l'acide sulfurique décrit par Zhuang, Quian et Wan dans Applied Surface Science, 2010, 256 ; 5343-5348 « An alternative method to remove PEO-PPO-PEO template in organic-inorganic mesoporous nanocomposites by sulfuric acid extraction ». Ce procédé présente l'inconvénient d'altérer le tensioactif qui ne peut plus être recyclé. De plus, cette technique nécessite la mise en place d'un lourd et coûteux système de recyclage de l'acide.

Les travaux de Tanev et Pinnavaia (Mater. 1996, 8 (8) : 2068-2079) décrivent quant à eux un procédé d'extraction du tensioactif par solvant. L'extraction est facilitée lorsque le tensioactif est non ionique. Bien que ce procédé présente l'avantage de permettre le recyclage du tensioactif, l'élimination de celui-ci du matériau poreux reste incomplète. Ce procédé est donc généralement couplé à une calcination qui permet de libérer complètement la porosité du matériau synthétisé.

Enfin, Huang, Xu et Li (Separation and Purification Technology, 118 ; 120-126 «Effect of the polar modifiers on supercritical extraction efficiency for template removal from hexagonal mesoporous silica materials : solubility parameter and polarity considérations ») montrent qu'un fluide supercritique peut permettre d'extraire le tensioactif du matériau mésoporeux. Si ce procédé permet d'éliminer efficacement le tensioactif et de le conserver intact, sa mise en œuvre au niveau industriel est compliquée.

Les silices mésoporeuses de l'art antérieur doivent donc être soumises à un processus qui permet l'élimination du tensioactif. Ce processus est assez compliqué, énergivore et onéreux. Il existe ainsi un besoin pour un procédé de synthèse de silice mésoporeuse et une silice mésoporeuse dans lesquels l'élimination du tensioactif pourrait être évitée et/ou facilitée et/ou peu onéreuse.

La présente invention vise à fournir une solution à au moins l'un des problèmes mentionnés ci-dessus. L'invention fournit un procédé de synthèse de silice mésoporeuse et une silice mésoporeuse obtenue par le procédé. Le procédé et la silice sont tel que décrits dans les revendications.

### Résumé de l'invention

Dans un premier aspect, la présente invention fournit une silice mésoporeuse préparée en mélangeant les composants A et B tel que A est un tensioactif de la famille des saponines et B est un précurseur de silice.

Le précurseur de silice est représenté par la formule Si(OR₁)(OR₂)(OR₃)(OR₄) où R₁, R₂, R₃ et R₄ sont indépendamment choisis parmi les groupes hydroxyles, alkyles, glycols, trimethyl-1,2,3,4-tetrahydronaphthalen, 1,1,1,3,3,3-hexafluoropropan-2-yl, dimethylsilyl, trimethylsilyl, ethoxysilyl, tributoxysilyl, diethoxy(methoxy)silyl, trimethoxysilyl, ethoxy(dimethoxy)silyl, butoxy(dipropoxy)silyl, tripropoxysilyl, diethoxy(trimethylsilyloxy)silyl, ethoxy-bis(trimethylsilyloxy)silyl, methyl-bis(trimethylsilyloxy)silyl, butoxy-bis(trimethylsilyloxy)silyl, diethoxy(triethoxysilyloxy)silyl, dimethyl(vinyl)silyl, trimethylsilyloxy, (3-methylpentoxy)silyl, 4,7,7-trimethyl-3-bicyclo[2.2.1]heptanyl, 2,2,4-trimethyl-3-bicyclo[2.2.1]heptanyl, propan-2-yloxy-bis(trimethylsilyloxy)silyl, dibutoxy(trimethylsilyloxy)silyl, trimethyl trimethoxysilyl, dibutoxy(ethenyl)silyl, diethyl bis(trimethylsilyl), (butan-2-yloxy)silyl, diacetyloxy-[(2-methylpropan-2-yl)oxy]silyl, acetyloxy(diethoxy)silyl, 4-(dimethylamino)phenyl, 4-(dimethylamino)phenyl, 2-(diethylamino)ethyl, pyridin-3-yl, 2-methylpropan-2-yl)oxy, 2,2,3,3,4,4,5,5,6,6,7,7-dodecafluoroheptyl, trichloro-2-ethylbutoxy, cyclononyl, 1-methoxypropan-2-yl, 2-(2-methoxyethoxy)ethyl, 2-butoxyethyl, 2-ethoxyethyl, 2-methoxyethyl, acetyl, acetyloxy(dipropoxy)silyl, 5-methyl-2-propan-2-ylcyclohexyl, butan-2-yloxy, methylphenyl, cyclohexyl, 2-aminoethyl, phenyl, prop-2-enyl, 2-fluoroethyl, acétate ou trihydroxysilyloxy. La configuration R1=R2=R3=R4 est couverte par l'invention,
ou
par la formule xSiO₂:M_{y}O où M est un ou des atomes de métal, un ou des atomes de métal de transition, un ou des atomes de non métal, un méthylammonium, un actinide, y = 1 ou 2 ou 3 ou 4 et x est le rapport molaire SiO₂/M_{y}O.

Dans un deuxième aspect, la présente invention fournit une méthode de production de silice mésoporeuse dans laquelle des composants A et B sont mélangés dans un solvant comprenant de l'eau, tel que A est un tensioactif de la famille des saponines et B est un précurseur de silice représenté par la formule Si(OR₁)(OR₂)(OR₃)(OR₄) où R₁, R₂, R₃ et R₄ sont indépendamment choisis parmi les groupes hydroxyles, alkyles, glycols, trimethyl-1,2,3,4-tetrahydronaphthalen, 1,1,1,3,3,3-hexafluoropropan-2-yl, dimethylsilyl, trimethylsilyl, ethoxysilyl, tributoxysilyl , diethoxy(methoxy)silyl, trimethoxysilyl, ethoxy(dimethoxy)silyl, butoxy(dipropoxy)silyl, tripropoxysilyl, diethoxy(trimethylsilyloxy)silyl, ethoxy-bis(trimethylsilyloxy)silyl, methyl-bis(trimethylsilyloxy)silyl, butoxy-bis(trimethylsilyloxy)silyl, diethoxy(triethoxysilyloxy)silyl, dimethyl(vinyl)silyl, trimethylsilyloxy, (3-methylpentoxy)silyl, 4,7,7-trimethyl-3-bicyclo[2.2.1]heptanyl, 2,2,4-trimethyl-3-bicyclo[2.2.1]heptanyl, propan-2-yloxy-bis(trimethylsilyloxy)silyl, dibutoxy(trimethylsilyloxy)silyl, trimethyl trimethoxysilyl, dibutoxy(ethenyl)silyl, diethyl bis(trimethylsilyl), (butan-2-yloxy)silyl, diacetyloxy-[(2-methylpropan-2-yl)oxy]silyl, acetyloxy(diethoxy)silyl, 4-(dimethylamino)phenyl, 4-(dimethylamino)phenyl, 2-(diethylamino)ethyl, pyridin-3-yl, 2-methylpropan-2-yl)oxy, 2,2,3,3,4,4,5,5,6,6,7,7-dodecafluoroheptyl, trichloro-2-ethylbutoxy, cyclononyl, 1-methoxypropan-2-yl, 2-(2-methoxyethoxy)ethyl, 2-butoxyethyl, 2-ethoxyethyl, 2-methoxyethyl, acetyl, acetyloxy(dipropoxy)silyl, 5-methyl-2-propan-2-ylcyclohexyl, butan-2-yloxy, methylphenyl, cyclohexyl, 2-aminoethyl, phenyl, prop-2-enyl, 2-fluoroethyl, acétate, trihydroxysilyloxy. La configuration R1=R2=R3=R4 est couverte par l'invention,
ou
par la formule xSiO₂:M_{y}O où M est un ou des atomes de métal, un ou des atomes de métal de transition, un ou des atomes de non métal, un méthylammonium, un actinide, y = 1 ou 2 ou 3 ou 4 et x est le rapport molaire SiO₂/M_{y}O.

La silice mésoporeuse de l'invention peut être utilisée dans les domaines de l'alimentation humaine ou animale, de la nutrition humaine ou animale, de l'industrie pharmaceutique, de l'industrie cosmétique.

La méthode et la silice de l'invention présentent plusieurs avantages par rapport aux silices de l'art antérieur et leurs méthodes de production. La méthode de l'invention permet de produire une silice comprenant un tensioactif naturel et de grade alimentaire, en particulier un tensioactif appartenant à la famille des saponines. La silice comprenant le tensioactif naturel peut être utilisée pour diverses applications. L'élimination totale du tensioactif n'est pas nécessairement requise et sa présence au niveau de la silice n'a aucun effet négatif sur les diverses applications envisagées pour la silice.

Le fait que la silice puisse être utilisée sans élimination totale du tensioactif permet de (i) maintenir les propriétés de la silice et éviter toutes altérations de la silice pouvant être occasionnées par les traitements, notamment par calcination, visant à l'élimination du tensioactif ; (ii) considérablement réduire le temps et/ou le coût de préparation de la silice ; et (iii) préserver l'environnement en évitant l'utilisation de produits chimiques ou de méthodes énergivores pour l'élimination du tensioactif.

Le maintien des propriétés de la silice (point (i) ci-dessus) permet de garantir l'efficacité, et l'optimisation des applications faisant recours à la silice de l'invention.

Dans le cas où le tensioactif doit être éliminé, la méthode et la silice de l'invention restent avantageuses par rapport aux silices et méthodes de l'art antérieur. En effet, le procédé et la silice de l'invention permettent une élimination du tensioactif par de simple lavage par de l'eau ou par une solution hydroalcoolique. Le fait d'utiliser un tensioactif de grade alimentaire autorise la présence résiduelle de tensioactif. Lorsque des tensioactifs plus toxiques sont utilisés, il devient nécessaire de supprimer toute trace de tensioactifs, notamment pour les applications alimentaires, médicales voire cosmétiques, et il est alors souvent nécessaire d'effectuer une étape de calcination à haute température ce qui n'est pas le cas pour la présente invention.

### Brève description des figures

**Figure 1** montre une photographie effectuée par microscope électronique Philips XL 30 ESEM représentant des particules de silices.
**Figure 2** graphique montrant l'isotherme d'adsorption de l'azote liquide à 77K pour des particules obtenues dans l'exemple 1.

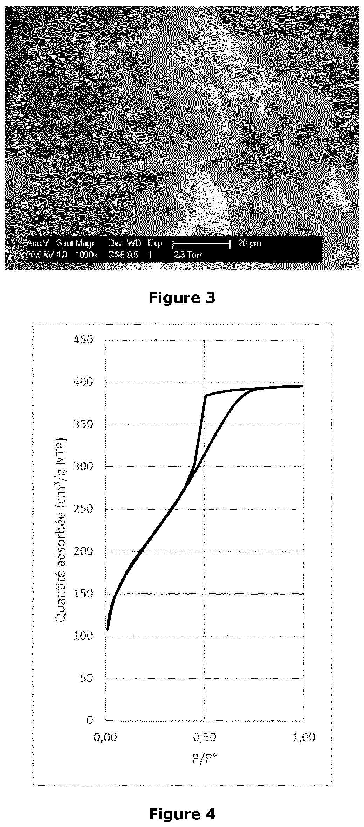

**Figure 3** montre une photographie représentant des particules de silices obtenues dans l'exemple 2. La photographie a été effectuée par microscope électronique Philips XL 30 ESEM.
**Figure 4** graphique montrant l'isotherme d'adsorption de l'azote liquide à 77K pour des particules obtenues dans l'exemple 2.
**Figure 5** montre une photographie représentant des particules de silices obtenues dans l'exemple 3 effectuée par microscope électronique Philips XL 30 ESEM.
**Figure 6** montre une photographie représentant des particules de silices obtenues dans l'exemple 3 et notamment la porosité des particules obtenues effectuée par microscope électronique Philips XL 30 ESEM.

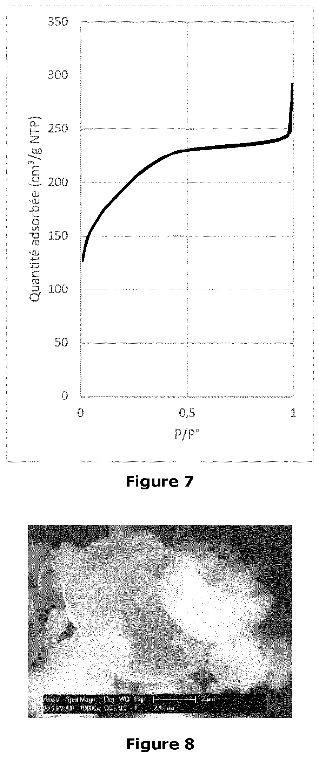

**Figure 7** graphique montrant l'isotherme d'adsorption de l'azote liquide à 77K pour des particules obtenues dans l'exemple 3.
**Figure 8** Photographie représentant des particules de silices obtenues dans l'exemple 5 effectuée par microscope électronique Philips XL 30 ESEM.

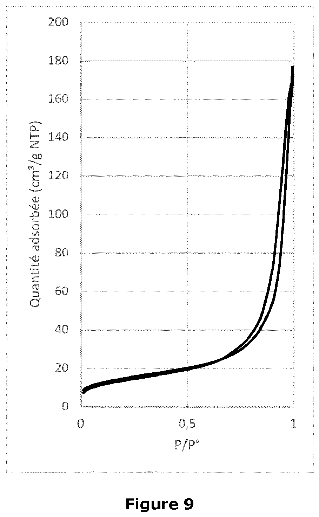

**Figure 9** graphique montrant l'isotherme d'adsorption de l'azote liquide à 77K pour des particules obtenues dans l'exemple 4.

### Description de l'invention

La présente invention concerne une méthode pour la production de silice mésoporeuse. La méthode est caractérisée par l'utilisation d'au moins un composé naturel et de grade alimentaire, notamment de la famille des saponines comme agent de structuration. La méthode peut être une synthèse en sol-gel ou en spray drying. L'invention concerne également la silice mésoporeuse obtenue par le biais dudit procédé.

Sauf mention contraire, tous les termes utilisés dans la révélation de l'invention, y compris les termes techniques et scientifiques, ont la signification telle que généralement comprise par l'homme de l'art. Des définitions de terme sont incluses pour mieux apprécier l'enseignement de la présente invention.

Tels qu'utilisés dans ce document, les termes suivants ont les significations suivantes :
« Un », « une », et « le » tels qu'utilisés dans ce document réfèrent aux référents tant singuliers que pluriels à moins que le contexte ne dise clairement autre chose. À titre d'exemple, « un compartiment » se réfère à un ou plus d'un compartiment.

« Environ » tel qu'utilisé dans ce document, fait référence à une valeur mesurable comme un paramètre, une quantité, une durée temporelle, et ainsi de suite, signifie englober des variations de +/- 20 % ou moins, de préférence +/- 10 % ou moins, plus de préférence +/- 5 % ou moins, même plus de préférence +/- 1 % ou moins et toujours plus de préférence +/- 0,1 % ou moins de et par rapport à la valeur spécifiée, pour autant que de telles variations soient appropriées pour fonctionner dans l'invention révélée. Cependant, on comprendra que la valeur à laquelle le modificateur « environ » se réfère soit également révélée de manière spécifique.

« Comprendre », « comprenant » et « comprend » et « composé de » tels qu'utilisés dans ce document sont synonymes de « incluent », « incluant », « inclut » ou « contiennent », « contenant », « contient » et sont des termes inclusifs ou ouverts qui spécifient la présence de ce que suit par exemple un composant et n'excluent pas ou n'écartent pas la présence de composants, particularités, éléments, organes, étapes, supplémentaires, non cités, connus dans la technique ou révélés en son sein.

La citation de plages numériques par des limites inclut tous les nombres et fractions englobés dans cette plage, aussi bien que les limites citées.

La silice mésoporeuse de l'invention est aussi désignée par les termes silice green ou silice verte.

Les termes « agent de structuration » et « tensioactif » sont utilisés comme synonymes.

Dans un premier aspect, la présente invention fournit une silice mésoporeuse préparée à partir des composants A et B tel que A est un tensioactif de la famille des saponines et B est un précurseur de silice. La silice est obtenue en mélangeant les composants A et B. Le précurseur peut être soluble ou non soluble dans l'eau.

Dans un mode de réalisation préféré, le précurseur de silice est représenté par la formule Si(OR₁)(OR₂)(OR₃)(OR₄) où R₁, R₂, R₃ et R₄ sont indépendamment choisis parmi les groupes hydroxyles, alkyles, glycols, trimethyl-1,2,3,4-tetrahydronaphthalen, 1,1,1,3,3,3-hexafluoropropan-2-yl, dimethylsilyl, trimethylsilyl, ethoxysilyl, tributoxysilyl, diethoxy(methoxy)silyl, trimethoxysilyl, ethoxy(dimethoxy)silyl, butoxy(dipropoxy)silyl, tripropoxysilyl, diethoxy(trimethylsilyloxy)silyl, ethoxy-bis(trimethylsilyloxy)silyl, methyl-bis(trimethylsilyloxy)silyl, butoxy-bis(trimethylsilyloxy)silyl, diethoxy(triethoxysilyloxy)silyl, dimethyl(vinyl)silyl, trimethylsilyloxy, (3-methylpentoxy)silyl, 4,7,7-trimethyl-3-bicyclo[2.2.1]heptanyl, 2,2,4-trimethyl-3-bicyclo[2.2.1]heptanyl, propan-2-yloxy-bis(trimethylsilyloxy)silyl, dibutoxy(trimethylsilyloxy)silyl, trimethyl trimethoxysilyl, dibutoxy(ethenyl)silyl, diethyl bis(trimethylsilyl), (butan-2-yloxy)silyl, diacetyloxy-[(2-methylpropan-2-yl)oxy]silyl, acetyloxy(diethoxy)silyl, 4-(dimethylamino)phenyl, 4-(dimethylamino)phenyl, 2-(diethylamino)ethyl, pyridin-3-yl, 2-methylpropan-2-yl)oxy, 2,2,3,3,4,4,5,5,6,6,7,7-dodecafluoroheptyl, trichloro-2-ethylbutoxy, cyclononyl, 1-methoxypropan-2-yl, 2-(2-methoxyethoxy)ethyl, 2-butoxyethyl, 2-ethoxyethyl, 2-methoxyethyl, acetyl, acetyloxy(dipropoxy)silyl, 5-methyl-2-propan-2-ylcyclohexyl, butan-2-yloxy, methylphenyl, cyclohexyl, 2-aminoethyl, phenyl, prop-2-enyl, 2-fluoroethyl, acétate, ou trihydroxysilyloxy. La configuration R1=R2=R3=R4 est couverte par l'invention.

Les groupes alkyl sont de préférence choisis parmi les groupes méthyle et éthyle. Il est préférable que toutes les liaisons O-R soient hydrolysables. Le précurseur de silice préféré de formule Si(OR)₄ est l'orthosilicate de tétraéthyle et/ou l'orthosilicate de tétraméthyle.

Suivant un mode de réalisation de l'invention, le précurseur de silice est représenté par la formule xSiO₂:M_{y}O où M est un ou des atomes de métal, un ou des atomes de métal de transition, un ou des atomes de non métal, un méthylammonium, un actinide, y = 1 ou 2 ou 3 ou 4 et x est le rapport molaire SiO₂/M_{y}O. L'atome de métal peut être alcalin dans (y=2) ou alcalino-terreux (y=1). Le précurseur de silice de formule xSiO₂:M_{y}O peut être choisis dans le groupe comprenant l'orthosilicate, le métasilicate de sodium, de potassium ou de calcium.

Dans un mode de réalisation préféré, l'agent de structuration appartient à la famille des saponines. Les saponines sont des molécules amphiphiles naturellement produites par certaines plantes ou animaux et présentant des propriétés tensioactives. Les sources les plus importantes de saponines utilisées dans l'industrie alimentaire et en cosmétique sont l'arbre Quillaja saponaria Molina, le Yucca schidigera, et l'arbuste du Sud-Est asiatique Camellia sinensis, connue sous le nom de « tea plant».

Les inventeurs ont découvert que le mécanisme d'auto-assemblage coopératif peut se réaliser en présence de tensioactifs naturels de type saponine. Il apparait que, comme dans le cas des tensioactifs industriels « classiques », les saponines s'agrègent pour former, avec le précurseur de silice une phase hybride sur laquelle la synthèse de matériau mésoporeux va se produire.

Les saponines utilisées dans le cadre de cette invention sont des glycosides triterpénoïdes dont la structure principale (I) aglycone (sapogénine) peut prendre les formes suivantes : dammarenediols (dammaranes), cucurbitadienol (cucurbitanes), hopanol (hopanes), lanostérols (lanostanes), tirucalladienol (tirucallanes), β-amyrin (oléananes), ∞-amyrin (ursanes), taraxastérols (taraxastéranes), lupéol (lupanes).

Des radicaux méthyl, des fonctions carboxylique, aldéhyde ou alcool ; des atomes d'hydrogène, des groupements hydroxyl ; ainsi que des chaines osidiques simples ou ramifiées sont par ailleurs attachées au squelette aglycone. Au sein des chaines osidiques, les sucres suivants sont préférés : D-glucose, L-rhamnose, D-galactose, D-acide glucuronique, L-arabinose, D-xylose, D-fructose, D-adipose, D-fucose. Dans le cas où l'aglycone comprend une chaine osidique, on parlera de monodesmoside et dans le cas où deux chaines osidiques sont liées au squelette aglycone on parlera de bidesmoside.

Dans un mode de réalisation préféré, la saponine utilisée présente la structure oléanane suivante telle que R1 et R4 sont des groupements méthyl, R2 est un atome d'hydrogène et R3 est un groupement carboxylique.

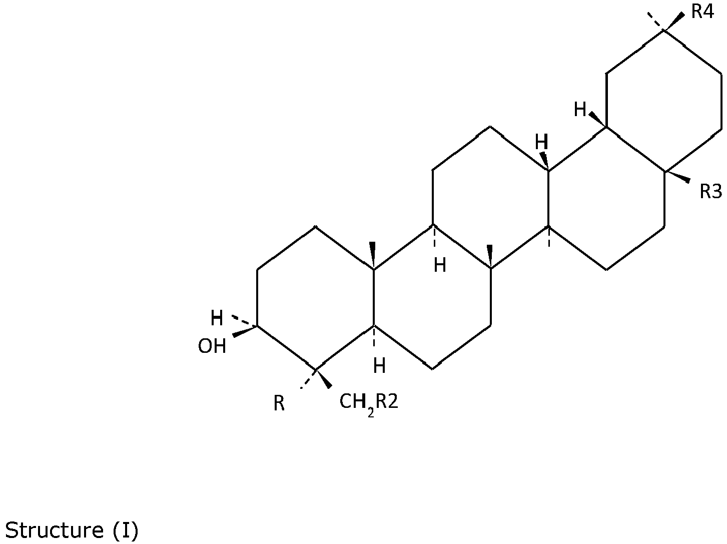

Suivant un autre mode de réalisation avantageux, l'agent de structuration contient une ou plusieurs des saponines suivantes : saponine 1, saponine 2, saponine 3, saponine 4, saponine 5, saponine 6, saponine 7, saponine 8, saponine 9, saponine 10, saponine 19, saponine 20a, saponine 20b, saponine 21a, saponine 21b, saponine 22a, saponine 22b, saponine 23, saponine S7, saponine S8, saponine S9, saponine S10, saponine S11, saponine S12, Quillaja saponines 7, 17, 18 21 (aussi dénommées QA-7, QA-17, QA-18, QA-21).

Suivant un mode de réalisation de l'invention, les saponines utilisées présentent une structure principale aglycone stéroïdale de type spirostanol ou furostanol ou une structure glycoalkaloïde stéroïdale de type spirosolane ou solanidane.

Dans un mode de réalisation préféré, la silice mésoporeuse de l'invention comprend au maximum 30% en poids d'agent structurant par rapport au poids du solide, de préférence entre 1 et 20% et plus avantageusement entre 1 et 5% de poids d'agent structurant par rapport au solide. Ledit poids du solide fait référence à la somme du poids de la silice et de l'agent structurant.

Dans un mode de réalisation préféré, la silice comprend des mésopores dont le diamètre moyen minimal est 1 nm. Le diamètre des pores a été calculé à partir des isothermes de sorption d'azote à température de l'azote liquide (77K) selon la méthode de Barrett, Joyner et Halenda (BJH). Le diamètre des mésopores de la silice, établi à partir de l'isotherme de la sorption d'azote à 77K, est de 2, 3, 4, 5, 6, 7, 8, 9, 10, 11, 12, 13, 14, 15, 16, 17, 18, 19, 20, 21, 22, 23, 24 ou 25 nm ou toute valeur comprise entre les valeurs précitées.

Dans un mode de réalisation préféré, la surface spécifique de la silice, calculée à partir des isothermes de sorption d'azote à température de l'azote liquide (77K) par la théorie Brunauer, Emmett et Teller (BET), est au moins 20 m²/g, 40 m²/g, 50 m²/g, ou 60 m²/g. La surface spécifique est au maximum 800 m²/g, 900 m²/g, 1000 m²/g ou 1500 m²/g ou toute valeur comprise entre les valeurs précitées.

Dans un mode de réalisation préféré, le volume poreux de la silice a été calculé à partir des isothermes de sorption d'azote à température de l'azote liquide (77K) par la méthode de Barrett, Joyner et Halenda (BJH). Ledit volume poreux est au moins de 0,1 cm³/g, 0,2 cm³/g, 0,4 cm³/g, 0,5 cm³/g et au maximum 0,6 cm³/g, 0,8 cm³/g, 1 cm³/g, 1,2 cm³/g, 1,5 cm³/g ou toute valeur comprise entre les valeurs précitées.

Dans un deuxième aspect, la présente invention fournit une méthode de production de silice mésoporeuse dans laquelle des composants A et B sont mélangés dans un solvant comprenant de l'eau, tel que A est un tensioactif naturel de la famille des saponines et B est un précurseur de silice. Les composants A et B sont tels que décrits précédemment. La méthode de production est réalisée à partir d'une solution micellaire de tensioactif dans un solvant comprenant de l'eau dans lequel est ajoutée la source de silice. Ladite source de silice est de préférence ajoutée goutte à goutte. De préférence, le solvant est l'eau ou une solution hydroalcoolique. Le tensioactif est tel que décrit ci-dessus.

Dans un mode de réalisation préféré, le précurseur de silice est représenté par la formule Si(OR)₄ où R est un groupe alkyl, ou par la formule xSiO₂:M_{y}O où M est un atome de métal, y = 1 ou 2 et x est le rapport molaire SiO₂/M_{y}O. Le précurseur de silice est tel que décrit précédemment.

Dans un mode de réalisation préféré, le tensioactif est ajouté au solvant en quantité suffisante pour obtenir une concentration micellaire comprise entre 0.1 et 1000 fois la concentration micellaire critique. De préférence, le tensioactif est ajouté au solvant en quantité suffisante pour obtenir une concentration micellaire comprise entre 10 et 900 fois, 20 et 800 fois, 30 et 700 fois, 40 et 600 fois, 50 et 500 fois, 60 et 400 fois, 70 et 300 fois, 80 et 200 fois, 90 et 100 fois la concentration micellaire critique ou toutes valeurs comprises entre les valeur précitées.

Dans un mode de réalisation préféré, le précurseur de silice est ajouté au solvant en quantité suffisante pour que le rapport entre la quantité de précurseur de silice et la quantité d'agent de structuration soit compris entre 0.1 et 50. Le précurseur de silice est préférentiellement ajouté goutte à goutte au solvant.

Dans un mode de réalisation préféré, la température de la solution obtenue suite à l'ajout du précurseur de silice au solvant est maintenue entre 15 et 35°C, de préférence entre 20 et 25°C. Le pH de la solution de tensio-actif est au maximum de 7. La solution peut avoir un pH acide qui est obtenu par l'ajout de solutions et/ou molécules adéquates tel que l'HCl. Le pH du solvant peut être 0, 1, 2, 3, 4, 5, 6, 7 ou toute valeur comprise entre les valeurs précitées. Le pH du solvant est préférentiellement de 1.

Dans un mode de réalisation préféré, le mélange est laissé au repos pendant au moins 5 heures et au maximum 24 heures à une température minimale de 20°C, le repos est suivi par une étape de filtration permettant l'obtention d'un produit solide a. Le mélange peut aussi être agité pendant au moins 10 minutes à une température minimale de 20°C, l'agitation est suivie par une évaporation du mélange obtenant ainsi un produit solide b. Le produit a et/ou le produit b est ensuite soumis à au moins un lavage par une solution d'éthanol, d'eau ou une solution hydroalcoolique à pression atmosphérique. Le produit lavé est ensuite soumis à au moins une étape de séchage sous pression réduite de 0,1 à 20 mm Hg.

Dans un mode de réalisation préféré, la méthode de production de la silice mésoporeuse comprend les étapes suivantes :
- L'introduction de saponines dans de l'eau acidifiée en une quantité nécessaire et suffisante pour obtenir une concentration micellaire comprise entre 0.1 et 1000, de préférence entre 1 et 1000 fois la CMC ;
- La mise en solution des saponines par le maintien de la solution à une température comprise en 15 et 35 °C ;
- L'addition au goutte à goutte d'un précurseur de silice de telle sorte que le rapport entre la quantité de précurseur de silice et la quantité d'agent de structuration soit compris entre 0.5 et 50, de préférence entre 1 et 50 ;
- Le vieillissement du mélange sans agitation pendant un laps de temps variant de 10 à 100 heures et préférentiellement de 10 à 20 heures à une température comprise entre 20 et 70°C ;
- La filtration du produit du vieillissement sur Büchner ;
- La réalisation de lavages successifs à pression atmosphérique et à température contrôlée par une solution d'éthanol à 40%vol ; et
- Le séchage du produit sous pression réduite.

Dans un mode de réalisation préféré, la méthode de production de la silice mésoporeuse comprend les étapes suivantes :
- L'introduction de saponines dans de l'eau acidifiée ou non en un volume nécessaire et suffisant pour obtenir une concentration micellaire comprise entre 0.1 et 1000, de préférence entre 1 et 1000 fois la CMC ;
- La mise en solution des saponines par le maintien de la solution à une température comprise entre 15 et 35 °C ;
- L'addition au goutte à goutte d'un précurseur de silice de telle sorte que le rapport entre la quantité de précurseur de silice et la quantité d'agent de structuration soit compris entre 0.5 et 50, de préférence entre 1 et 50 ;
- Le vieillissement du mélange avec ou sans agitation, pendant 30 minutes à 72 heures à une température comprise entre 20 et 30°C ;
- La mise en œuvre de l'évaporation par voie aérosol. Le mélange précurseur-surfactant est aspiré au niveau du spray-dryer et est poussé au travers d'une buse deux fluides ou ultrasonore présentant un orifice de 0,15 à 1,2 mm afin de générer des gouttelettes d'environ 20 µm. La température en entrée de colonne est comprise entre 120 et 250°C et de préférence entre 140 et 180°C. Un flux d'air d'une puissance inférieure à 1 m³/min ainsi qu'une température en tête de colonne adaptés permettent, 1) l'évaporation du solvant aqueux, 2) l'auto-assemblage des micelles de surfactant et 3) la condensation du précurseur de silice.
- La poudre obtenue, de couleur blanchâtre, est lavée comme décrit précédemment.

La méthode de l'invention permet d'obtenir des taux de rendement élevés de l'ordre de 60 à 90% par rapport à la masse attendue en solide. Ladite masse fait référence à la somme de la masse de la silice et de l'agent structurant.

Le procédé de synthèse de silice mésoporeuse selon l'invention est caractérisé par le mélange et la mise en réaction d'un précurseur de silice et d'un agent de structuration naturel. L'invention est également caractérisée par le fait que, compte tenu des propriétés chimiques et biologiques de l'agent de structuration, son élimination est rendue facultative. Avantageusement, l'agent de structuration n'est pas éliminé ce qui permet d'accélérer le procédé industriel et de diminuer le coût de l'industrialisation.

Dans le cas où l'élimination de l'agent de structuration et donc de la saponine est envisagée ou requise, celui-ci peut être éliminé par lavage à l'eau subcritique ou par lavages successifs à l'eau ou par un mélange eau/éthanol à 40% vol sans avoir nécessairement recours à une étape de calcination. La saponine extraite pourrait être utilisée pour la production d'autres silices mésoporeuses et/ou pour d'autres applications connues par l'homme de l'art.

### Exemples

### Exemple 1 :

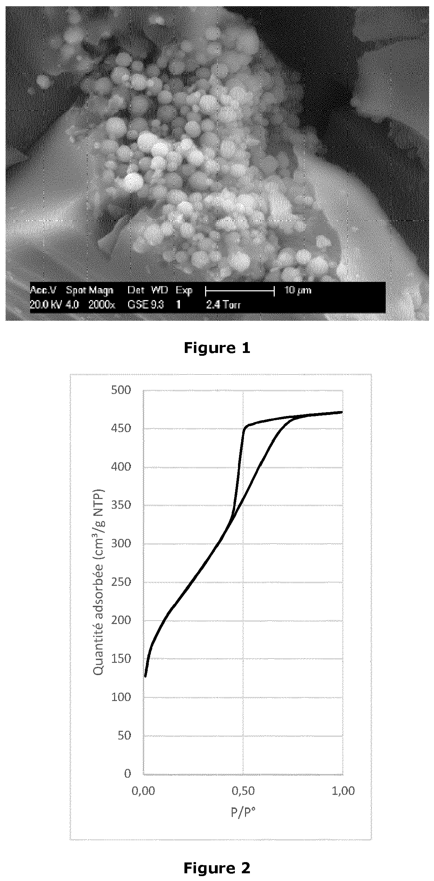

De l'acide chlorhydrique 12 N est ajouté à 115 ml d'eau de telle sorte que le pH soit compris entre 1 et 2. Une quantité de saponine (ABCR) permettant d'obtenir 10 fois la concentration micellaire critique est dissoute sous agitation à 25°C dans ce volume d'eau acidifiée. Une quantité de tétraéthoxysilane permettant d'obtenir un rapport précurseur / agent de structure de 50 est alors ajoutée sous agitation au goutte à goutte à raison de 0,3 grammes par minute. Le mélange obtenu a un pH compris entre 1 et 2. Le mélange est vieilli sans agitation pendant 16 heures à 65°C. Le gel obtenu est filtré sur Büchner, lavé plusieurs fois avec une solution d'éthanol à 40% vol avant d'être séché sous pression réduite à 40°C. La poudre obtenue est blanche. La silice mésoporeuse « green » récupérée présente une surface spécifique déterminée par la méthode du BET de 850 m²g⁻¹, un volume poreux de 0,7 cm³g⁻¹ et des mésopores de 35 Å. La forme des particules obtenues regroupe sphères et blocs de silices (**FIG.1** prise par microscope électronique PHILIPS XL 30 ESEM).

### Exemple 2 :

1.5 g de la silice mésoporeuse obtenue selon l'exemple 1 ont été calcinés à 500 °C pendant 4 heures. La poudre obtenue est blanche. La silice mésoporeuse présente une surface spécifique de 750 m²g⁻¹, un volume poreux de 0,6 cm³g⁻¹ et des mésopores de 32 Å.

### Exemple 3 :

Une quantité de saponine (Q-Naturale 200- Desert King) présentant un contenu en saponines triterpéniques de 67 à 73 % (poids sec) est dissoute sous agitation à 25°C dans un volume d'eau acidifiée permettant d'obtenir une concentration correspondant à 10 fois la concentration micellaire critique. Une quantité de tétraéthoxysilane permettant d'obtenir un rapport précurseur / agent de structure de 10 est alors ajouté sous agitation au goutte à goutte à raison de 0,3 grammes par minute. Le mélange obtenu a un pH compris entre 1 et 2. Le mélange est vieilli sans agitation pendant 12 à 16 heures à température ambiante. Le sol ainsi obtenu est introduit dans un atomiseur (spray dryer) (ProCept R&D spray dryer - Belgique) et pulvérisé grâce à une buse deux fluides présentant un orifice de 0,4 mm sous une pression d'air comprimé de 6 bars. La quantité de solution à atomiser est de 200 ml. Les conditions opératoires de l'atomiseur (spray dryer) sont les suivantes :
- La température de l'air entrant est fixée à 180°C
- La température en sortie de colonne est de 60°C
- La vitesse d'atomisation est de 7L.min⁻¹
2,24 grammes de poudre blanche de silice mésoporeuse présentant une densité apparente très faible sont collectés.

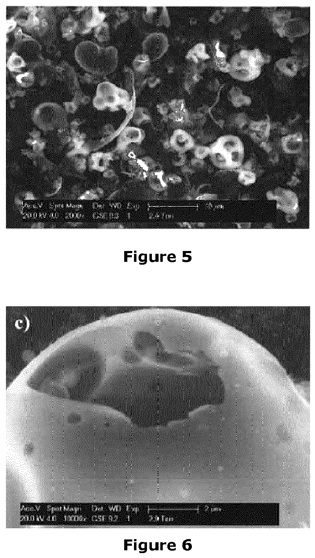

La poudre obtenue est observée en microscopie électronique et l'analyse montre la présence de particules en forme de cuvette ce qui est caractéristique des synthèses en spray drying (Waldron K et al. Formation of monodisperse mesoporous silica microparticles via spray drying. Journal of Colloïd and Interface Science 418 (2014) 225-233). Le matériel synthétisé présente une surface spécifique de 648 m²g⁻¹, un volume poreux de 0,38 cm³ g⁻¹ et des mésopores de 3,2 nm. La **Fig. 5****,** enregistrée à partir d'un microscope électronique PHILIPS XL 30 ESEM, montre la forme des particules obtenues. Une image montrant la porosité est représentée sur la **Fig. 6** enregistrée à partir d'un microscope électronique PHILIPS XL 30 ESEM.

### Exemple 4 :

Une quantité de saponine (Q-Naturale 200- Desert King) présentant un contenu en saponines triterpéniques de 67 à 73 % (poids sec) est dissoute sous agitation à 25°C dans un volume d'eau acidifiée permettant d'obtenir une concentration correspondant à 10 fois la concentration micellaire critique. Une quantité de métasilicate de potassium permettant d'obtenir un rapport précurseur / agent de structure de 10 est alors ajouté sous agitation au goutte à goutte à raison de 0,3 grammes par minute. Le mélange obtenu a un pH compris entre 1 et 2. Le mélange est vieilli sans agitation pendant 12 à 16 heures à température ambiante. Le sol ainsi obtenu est introduit dans un atomiseur (spray dryer) (ProCept R&D spray dryer - Belgique) et pulvérisé grâce à une buse deux fluides présentant un orifice de 0,4 mm sous une pression d'air comprimé de 6 bars. La quantité de solution à atomiser est de 200 ml. Les conditions opératoires de l'atomiseur (spray dryer) sont les suivantes :
- La température de l'air entrant est fixée à 180°C
- La température en sortie de colonne est de 60°C
- La vitesse d'atomisation est de 7L.min⁻¹
- 3,19 gr de poudre blanche de silice mésoporeuse présentant une densité apparente très faible sont collectés.

La poudre blanche obtenue est lavée à l'eau puis est observée en microscopie électronique sur un microscope Philips XL30 ESEM. L'analyse montre la présence de particules sphériques ainsi que de particules de forme moins bien définie.

Le matériel synthétisé présente une surface spécifique de 50 m²g⁻¹, un volume poreux de 0,23 cm³g⁻¹ et des mésopores de 19 nm.

## Revendications

1. Une silice mésoporeuse préparée à partir des composants A et B tel que A est un tensioactif de la famille des saponines et B est un précurseur de silice, ladite silice comprend entre 1% et 30% en poids de tensioactif.

2. La silice mésoporeuse selon revendication 1 dans laquelle les mésopores ont une taille minimale de 1 nm, calculé à partir des isothermes de sorption d'azote à température de l'azote liquide (77K) selon la méthode de Barrett, Joyner et Halenda (BJH).

3. La silice mésoporeuse selon revendication 1 ou 2 dont le volume poreux est au moins 0,1 cm³/g, calculé à partir des isothermes de sorption d'azote à température de l'azote liquide (77K) selon la méthode de Barrett, Joyner et Halenda (BJH).

4. La silice mésoporeuse selon l'une des revendications 1-3 dans laquelle le tensioactif est de grade alimentaire.

5. Méthode de production de silice mésoporeuse dans laquelle des composants A et B sont mélangés dans un solvant comprenant de l'eau, tel que A est un tensioactif de la famille des saponines et B est un précurseur de silice.

6. Méthode selon la revendication 5 dans laquelle le précurseur de silice est représenté par la formule Si(OR₁)(OR₂)(OR₃)(OR₄) où R₁, R₂, R₃ et R₄ sont indépendamment choisis parmi les groupes alkyles, hydroxyles, glycols, trimethyl-l,2,3,4-tetrahydronaphthalen, 1,1,1,3,3,3-hexafluoropropan-2-yl, dimethylsilyl, trimethylsilyl, ethoxysilyl, tributoxysilyl, diethoxy(methoxy)silyl, trimethoxysilyl, ethoxy(dimethoxy)silyl, butoxy(dipropoxy)silyl, tripropoxysilyl, diethoxy(trimethylsilyloxy)silyl, ethoxy-bis(trimethylsilyloxy)silyl, methyl-bis(trimethylsilyloxy)silyl, butoxy-bis(trimethylsilyloxy)silyl, diethoxy(triethoxysilyloxy)silyl, dimethyl(vinyl)silyl, trimethylsilyloxy, (3-methylpentoxy)silyl, 4,7,7-trimethyl-3-bicyclo[2.2.1]heptanyl, 2,2,4-trimethyl-3-bicyclo[2.2.1]heptanyl, propan-2-yloxy-bis(trimethylsilyloxy)silyl, dibutoxy(trimethylsilyloxy)silyl, trimethyl trimethoxysilyl, dibutoxy(ethenyl)silyl, diethyl bis(trimethylsilyl), (butan-2-yloxy)silyl, diacetyloxy-[(2-methylpropan-2-yl)oxy]silyl, acetyloxy(diethoxy)silyl, 4-(dimethylamino)phenyl, 4-(dimethylamino)phenyl, 2-(diethylamino)ethyl, pyridin-3-yl, 2-methylpropan-2-yl)oxy, 2,2,3,3,4,4,5,5,6,6,7,7-dodecafluoroheptyl, trichloro-2-ethylbutoxy, cyclononyl, 1-methoxypropan-2-yl, 2-(2-methoxyethoxy)ethyl, 2-butoxyethyl, 2-ethoxyethyl, 2-methoxyethyl, acetyl, acetyloxy(dipropoxy)silyl, 5-methyl-2-propan-2-ylcyclohexyl, butan-2-yloxy, methylphenyl, cyclohexyl, 2-aminoethyl, phenyl, prop-2-enyl, 2-fluoroethyl, acétate, ou trihydroxysilyloxy
ou
par la formule xSiO₂:M_{y}O où M est un ou des atomes de métal, un ou des atomes de métal de transition, un ou des atomes de non métal, un méthylammonium, un actinide, y = 1 ou 2 ou 3 ou 4 et x est le rapport molaire SiO₂/M_{y}O.

7. Méthode selon revendications 5 et/ou 6 dans laquelle le tensioactif est ajouté au solvant en quantité suffisante pour obtenir une concentration micellaire comprise entre 1 et 1000 fois la concentration micellaire critique.

8. Méthode selon une des revendications 5-7 dans laquelle le précurseur de silice est ajouté au solvant en quantité suffisante pour que le rapport entre la quantité de précurseur de silice et la quantité du tensioactif soit compris entre 0.5 et 50.

9. Méthode selon une des revendications 5-8 dans laquelle la température du solvant est maintenue entre 15 et 35°C.

10. Méthode selon une des revendications 5-9 dans laquelle le pH du solvant est au maximum de 7.

11. Méthode selon une des revendications 5-10 dans laquelle le mélange est laissé au repos pendant au moins 5 heures à une température minimale de 20°C, le repos est suivi par une étape de filtration obtenant ainsi un produit solide a.

12. Méthode selon une des revendications 5-11 dans laquelle le mélange est agité pendant au moins 10 min à une température minimale de 20°C, l'agitation est suivie par une évaporation et/ou une aspiration du mélange obtenant ainsi un produit solide b.

13. Méthode selon revendication 11 et/ou 12 dans laquelle le produit a et/ou le produit b est soumis à au moins un lavage par une solution d'éthanol à pression atmosphérique.

14. Méthode selon la revendication 13 dans laquelle le produit lavé est soumis à au moins une étape de séchage sous pression.

15. Utilisation de la silice mésoporeuse telle que décrite dans les revendications 1-4 pour des utilisations dans les domaines de l'alimentation humaine ou animale, des médicaments à usage humain ou animal, des cosmétiques, des biocides.
